# EUROPEAN PATENT SPECIFICATION

(11) **EP 2 870 932 B1**
(45) Date of publication and mention of the grant of the patent: **20.09.2017**
(21) Application number: 14192323.5
(22) Date of filing: 07.11.2014
(51) Int. Cl.: A61B 17/072, A61F 2/00, A61B 17/115, A61L 15/24, A61L 15/58, A61B 17/00

(54) **Hybrid adjunct materials for use in surgical stapling**
Hybride Zusatzmaterialien für chirurgische Klammervorrichtung
Matières auxiliaires hybrides destinées à être utilisées dans un agrafage chirurgical

(30) Priority: 08.11.2013 US 201314074810
(43) Date of publication of application: 13.05.2015
(60) Divisional application: 17186328.5
(73) Proprietor: Ethicon LLC, 00969 Guaynabo (PR)
(72) Inventor: Shelton, IV Frederick E., Hillsboro, OH 45133 (US); Widenhouse, Tamara S. V., Clarksville, OH 45113 (US)
(74) Representative: Carpmaels & Ransford LLP

(56) References cited:
- EP-A1- 1 994 890
- EP-A2- 2 008 595
- WO-A1-2013/119365
- US-A1- 2012 083 835

## Description

### FIELD

The present invention relates to surgical instruments, and in particular to devices, and components thereof for cutting and stapling tissue.

### BACKGROUND

Surgical staplers are used in surgical procedures to close openings in tissue, blood vessels, ducts, shunts, or other objects or body parts involved in the particular procedure. The openings can be naturally occurring, such as passageways in blood vessels or an internal organ like the stomach, or they can be formed by the surgeon during a surgical procedure, such as by puncturing tissue or blood vessels to form a bypass or an anastomosis, or by cutting tissue during a stapling procedure.

Most staplers have a handle with an elongate shaft having a pair of movable opposed jaws formed on an end thereof for holding and forming staples therebetween. The staples are typically contained in a staple cartridge, which can house multiple rows of staples and is often disposed in one of the two jaws for ejection of the staples to the surgical site. In use, the jaws are positioned so that the object to be stapled is disposed between the jaws, and staples are ejected and formed when the jaws are closed and the device is actuated. Some staplers include a knife configured to travel between rows of staples in the staple cartridge to longitudinally cut and/or open the stapled tissue between the stapled rows.

While surgical staplers have improved over the years, a number of problems still present themselves. One common problem is that leaks can occur due to the staple forming holes when penetrating the tissue or other object in which it is disposed. Blood, air, gastrointestinal fluids, and other fluids can seep through the openings formed by the staples, even after the staple is fully formed. The tissue being treated can also become inflamed due to the trauma that results from stapling. Still further, staples, as well as other objects and materials that can be implanted in conjunction with procedures like stapling, generally lack some characteristics of the tissue in which they are implanted. For example, staples and other objects and materials can lack the natural flexibility of the tissue in which they are implanted. A person skilled in the art will recognize that it is often desirable for tissue to maintain as much of its natural characteristics as possible after staples are disposed therein.

In some instances, biologic materials have been used in conjunction with tissue stapling. However, the use of biologic materials presents a number of additional problems. For example, it can be difficult to maintain a location of the biologic material with respect to jaws of the stapler prior to and during staple ejection. It can also be difficult to keep the biologic material at a desired location at the surgical site after stapling is completed. Further, it can be difficult to manufacture the biologic material to a desired shape and thickness. Common plastic and molding manufacturing techniques are not generally conducive to the manufacture of thin biologic layers for use in conjunction with surgical staplers. The fragile nature of many biologic materials also makes them difficult to use with surgical staplers because they lack structural support.

EP2008595 describes multilayer structures including a porous layer and a nonporous layer. When a stapling apparatus is fired it forces at least one staple to pass through openings in the staple cartridge, at least one multilayer buttress, the tissue, and openings in the staple anvil to seal the tissue.

US2012/083835 describes a fastener cartridge which can comprise a support portion, a tissue thickness compensator positioned relative to the support portion, and a plurality of fasteners positioned within the support portion and/or the tissue thickness compensator which can be utilized to fasten tissue. In use, the fastener cartridge can be positioned in a first jaw of a surgical fastening device, wherein a second jaw, or anvil, can be positioned opposite the first jaw. To deploy the fasteners, a staple-deploying member is advanced through the fastener cartridge to move the fasteners toward the anvil. As the fasteners are deployed, the fasteners can capture at least a portion of the tissue thickness compensator therein along with at least a portion of the tissue being fastened.

Accordingly, there remains a need for improved devices and methods for stapling tissue, blood vessels, ducts, shunts, or other objects or body parts such that leaking and inflammation is minimized while substantially maintaining the natural characteristics of the treatment region. There further remains a need for improved implantable materials that include biologies.

### SUMMARY

The invention is defined in the appended claims. Implantable materials for use with end effectors like surgical stapling devices, and methods for using the same, are generally provided. In one exemplary embodiment, the implantable material is a hybrid adjunct material for use with a surgical stapler. The hybrid adjunct material can include both a biologic layer and a synthetic layer, with the synthetic layer having opposed first and second surfaces, the first surface being mated to the biologic layer, and the second surface being configured to mate to a surgical stapler. At least one of the biologic layer and the synthetic layer can be configured to form a seal around legs of a staple.

In some embodiments, the synthetic layer can include one or more openings formed in it. The openings can allow biologic material from the biologic layer to pass therethrough. The synthetic layer can be a non-permeable, synthetic absorbable layer. In alternative embodiments, the synthetic layer can be permeable.

The biologic layer can include at least one patient-derived material disposed in it. Further, in some embodiments, a self-sealing port can be included. The self-sealing port can be in fluid communication with the biologic layer, and can be configured to permit delivery of patient-derived material to the biologic layer. The biologic layer can be a permeable, bioabsorbable membrane. The biologic layer and the synthetic layer are configured to be snap-fit together.

In one exemplary embodiment of a staple cartridge assembly for use with a surgical stapler, the assembly can include a cartridge body, a biologic tissue membrane, and a synthetic layer. The cartridge body can have a plurality of staple cavities configured to seat staples therein, and the synthetic layer can be associated with the biologic tissue membrane such that the synthetic layer is configured to provide structural integrity to the membrane so as to allow the membrane to be securely coupled to the cartridge body. The membrane and the synthetic layer can be configured to be securely attached to tissue by staples in the cartridge.

In some embodiments, the synthetic layer can have one or more openings formed therein to allow biologic material of the biologic tissue membrane to pass through the synthetic layer and to tissue located adjacent to the synthetic layer. The synthetic layer can be permeable, or alternatively, it can be non-permeable. The synthetic layer can also include one or more protrusions that extend from the layer and are configured to engage the biologic tissue membrane. Further, in some embodiments, a self-sealing port can be included. The self-sealing port can be in fluid communication with at least one of the synthetic layer and the biologic tissue membrane, and can be configured to permit delivery of patient-derived material to the membrane.

A variety of techniques and components can be used to secure the location of the synthetic layer and the biologic tissue membrane with respect to the cartridge body. For example, at least one bracket can be configured to couple the biologic tissue membrane and the synthetic layer to the cartridge body. The bracket(s) can be coupled to an outer edge of the cartridge body and an outer edge of at least one of the membrane and the synthetic layer. The biologic tissue membrane and the synthetic layer are configured to be snap-fit together.

In other aspects, a method for stapling tissue is provided and includes attaching a biologic layer and a synthetic layer to at least one of a cartridge assembly and an anvil of an end effector, engaging tissue between the cartridge assembly and the anvil, and actuating the end effector to eject staples from the cartridge assembly into the tissue. Actuation can cause the staples to extend through the biologic and synthetic layers to maintain the biologic and synthetic layers at the surgical site. In some embodiments, the method can include injecting one or more patient-derived fluids into the biologic layer. The synthetic layer can include one or more openings formed in it such that material from the biologic layer can pass through the one or more openings of the synthetic layer and into the tissue engaged by the staples. In some embodiments, the biologic layer and the synthetic layer can be attached to the end effector by snap-fitting the biologic layer into the synthetic layer.

### BRIEF DESCRIPTION OF THE DRAWINGS

The invention will be more fully understood from the following detailed description taken in conjunction with the accompanying drawings, in which:
FIG. 1 is a perspective view of one exemplary embodiment of a surgical instrument having an attachment portion attached to a distal end thereof;
FIG. 2 is a perspective view of the surgical instrument of FIG. 1 with the attachment portion detached from a shaft of the instrument;
FIG. 3 is a perspective view of the attachment portion of FIG. 2 including at least one piece of adjunct material;
FIG. 4 is an exploded perspective view of the end effector of FIG. 3 with the adjunct material removed;
FIG. 5 is a detailed perspective view of a distal end of a staple cartridge for use with the end effector of FIG. 4;
FIG. 6 is a side cross-sectional view taken along the section line indicated in FIG. 5;
FIG. 7 is a bottom perspective view of the staple cartridge of FIG. 5;
FIG. 8 is a detailed perspective view of an actuation sled, pushers, and fasteners of the surgical instrument of FIG. 4;
FIG. 9 is a perspective view of another exemplary embodiment of an attachment portion for use a surgical instrument;
FIG. 10 is an exploded perspective view of an end effector of the attachment portion of FIG. 9;
FIG. 11 is an exploded view of a drive assembly for use with the end effector of FIG. 4;
FIG. 12 is a perspective view of a lower jaw of the end effector of FIG. 3;
FIG. 13 is a perspective view of an upper jaw of the end effector of FIG. 3, the upper jaw having an adjunct material associated therewith;
FIG. 14 is a perspective view of portions of the end effector of FIG. 2 including a retention member configured to releasably retain an adjunct material;
FIG. 15 is a perspective view of a lower jaw of the end effector of FIG. 10;
FIG. 16A is a perspective exploded view of one exemplary embodiment of a hybrid adjunct material and a lower jaw of an end effector;
FIG. 16B is a front cross-sectional view of the hybrid adjunct material of FIG. 16A in a non-exploded configuration and taken along line A-A, and further illustrating a second hybrid adjunct material coupled to an upper jaw of the end effector;
FIG. 17 is a perspective cross-sectional view of another exemplary embodiment of a first hybrid adjunct material coupled to a lower jaw of an end effector and a second hybrid adjunct material coupled to an upper jaw of the end effector;
FIG. 18 is a perspective view of yet another exemplary embodiment of a hybrid adjunct material coupled to a lower jaw of an end effector, with a portion of a biologic layer of the hybrid adjunct material removed for illustrative purposes;
FIG. 19 is a perspective view of one exemplary embodiment of a synthetic layer of a hybrid adjunct material coupled to a lower jaw of an end effector;
FIG. 20 is a perspective view of another exemplary embodiment of a synthetic layer of a hybrid adjunct material coupled to a lower jaw of an end effector, and further including a biologic layer configured to couple to the synthetic layer;
FIG. 21A is a perspective view of one exemplary embodiment of an attachment portion of a surgical instrument having a synthetic layer coupled to a lower jaw thereof;
FIG. 21B is a perspective view of the lower jaw of FIG. 21A and a biologic layer configured to couple to the synthetic layer;
FIG. 21C is a front cross-sectional view illustrating the biologic layer of FIG. 21B partially coupled to the synthetic layer;
FIG. 21D is a front cross-sectional view illustrating the biologic layer of FIG. 21B fully coupled to the synthetic layer.
FIG. 21E is a perspective view of the lower jaw of FIG. 21A with the biologic layer fully coupled to the synthetic layer and a portion of the biologic layer removed for illustrative purposes;
FIG. 22A is a perspective view of another exemplary embodiment of an attachment portion of a surgical instrument having a hybrid adjunct material coupled to an upper jaw thereof;
FIG. 22B is a perspective view of the upper jaw of FIG. 22A;
FIG. 23A is a perspective view of one exemplary embodiment of an upper jaw of an end effector having a biologic material coupled thereto;
FIG. 23B is a detailed schematic view of a surgical site illustrating a staple disposed both in tissue and in the biologic material of FIG. 23A;
FIG. 23C is a detailed schematic view of the surgical site of FIG. 23B illustrating the staple after it is fully formed;
FIG. 24A is a perspective view of another exemplary embodiment of an upper jaw of an end effector having two biologic layers coupled thereto, with a portion of a first biologic layer removed for illustrative purposes;
FIG. 24B is a detailed view of the two biologic layers of FIG. 24A;
FIG. 24C is a detailed schematic view of a surgical site illustrating a staple disposed both in tissue and through the two biologic layers of FIG. 24B;
FIG. 24D is a detailed schematic view of the surgical site of FIG. 24C illustrating the staple after it is fully formed;
FIG. 25A is a perspective view of one exemplary embodiment of a hybrid adjunct material having a snap-fit configuration between a biologic layer and a synthetic layer thereof;
FIG. 25B is a perspective view of another exemplary embodiment of a hybrid adjunct material having biologic layer laminated to a synthetic layer;
FIG. 25C is a perspective view of still another exemplary embodiment of a hybrid adjunct material having biologic materials imbibed in a synthetic layer;
FIG. 26A is a perspective view of one exemplary embodiment of packaging for a surgical instrument having a synthetic layer of a hybrid adjunct material associated therewith; and
FIG. 26B is a perspective view of one exemplary embodiment of packaging for a biologic layer, the biologic layer being configured to mate with the synthetic layer of FIG. 26A.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present disclosure. Further, in the present disclosure, like-numbered components of the various embodiments generally have similar features when those components are of a similar nature and/or serve a similar purpose.

Reference throughout the specification to "various embodiments," "some embodiments," "one embodiment," or "an embodiment," or the like, means that a particular feature, structure, or characteristic described in connection with the embodiment is included in at least one embodiment. Thus, appearances of the phrases "in various embodiments," "in some embodiments," "in one embodiment," or "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation. Such modifications and variations are intended to be included within the scope of the present disclosure.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical," "horizontal," "up," and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, a person skilled in the art will appreciate that the various methods and devices disclosed herein can be used in numerous surgical procedures and applications. Those skilled in the art will further appreciate that the various instruments disclosed herein can be inserted into a body in any way, such as through a natural orifice, through an incision or puncture hole formed in tissue, or through an access device, such as a trocar cannula. For example, the working portions or end effector portions of the instruments can be inserted directly into a patient's body or can be inserted through an access device that has a working channel through which the end effector and elongated shaft of a surgical instrument can be advanced.

It can be desirable to use one or more biologic materials and/or synthetic materials, collectively referred to herein as "adjunct materials," in conjunction with surgical instruments to help improve surgical procedures. A person skilled in the art may refer to these types of materials as buttress materials. While a variety of different end effectors can benefit from the use of adjunct materials, in some exemplary embodiments the end effector can be a surgical stapler. When used in conjunction with a surgical stapler, the adjunct material(s) can be disposed between and/or on jaws of the stapler, incorporated into a staple cartridge disposed in the jaws, or otherwise placed in proximity to the staples. When staples are deployed, the adjunct material(s) can remain at the treatment site with the staples, in turn providing a number of benefits. In some instances, the material(s) can be used to help seal holes formed by staples as they are implanted into tissue, blood vessels, and various other objects or body parts. Further, the materials can be used to provide tissue reinforcement at the treatment site. Still further, the materials can help reduce inflammation, promote cell growth, and otherwise improve healing.

Some particularly advantageous configurations of adjunct materials include both synthetic and biologic materials. The combination of both types of materials can result in the formation of a hybrid adjunct material. Hybrid adjunct materials allow for the beneficial features of synthetic material(s) and the beneficial features of biologic material(s) to be incorporated into a single adjunct material. Thus, while often biologic material can be difficult to shape into a desired shape and then held in that desired configuration, by using synthetic material in conjunction with the biologic material, the synthetic material can serve as a support structure for the biologic material. Accordingly, the benefits of biologic material, such as improved healing and tissue growth at the surgical site, can be provided with the stability afforded by synthetic material.

### SURGICAL STAPLING INSTRUMENT

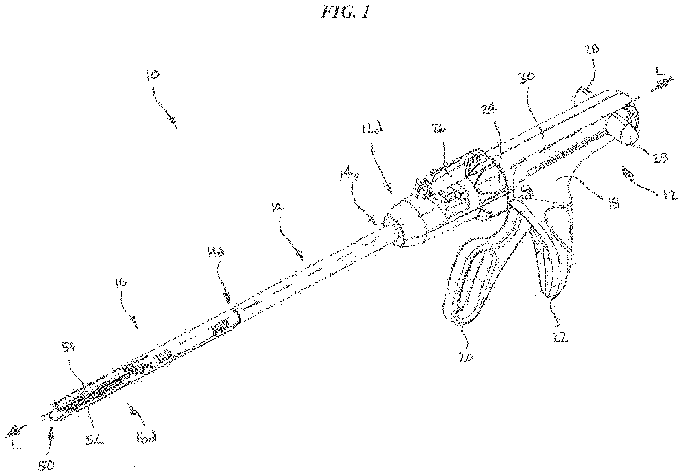

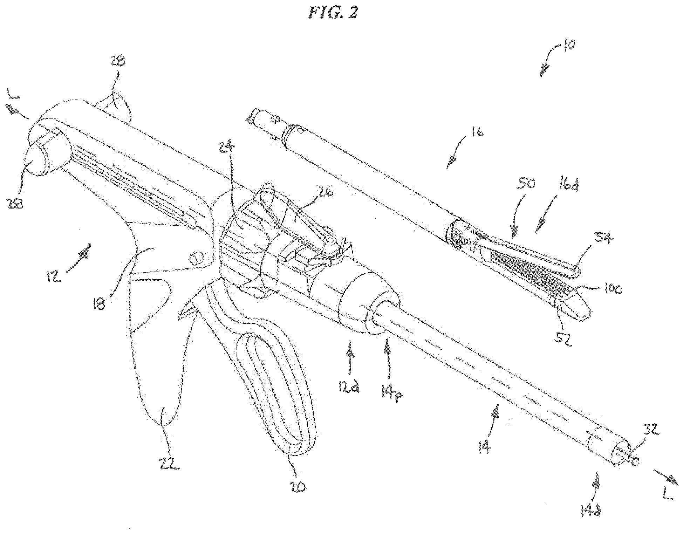

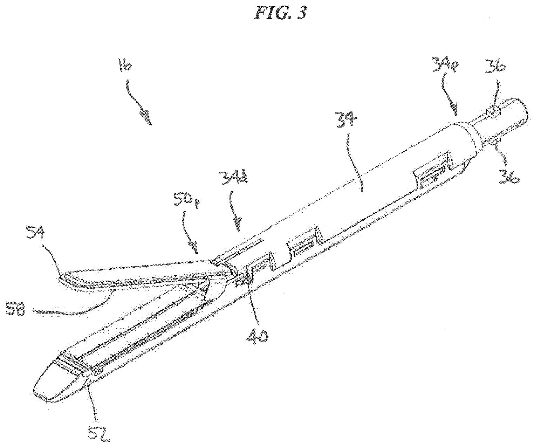

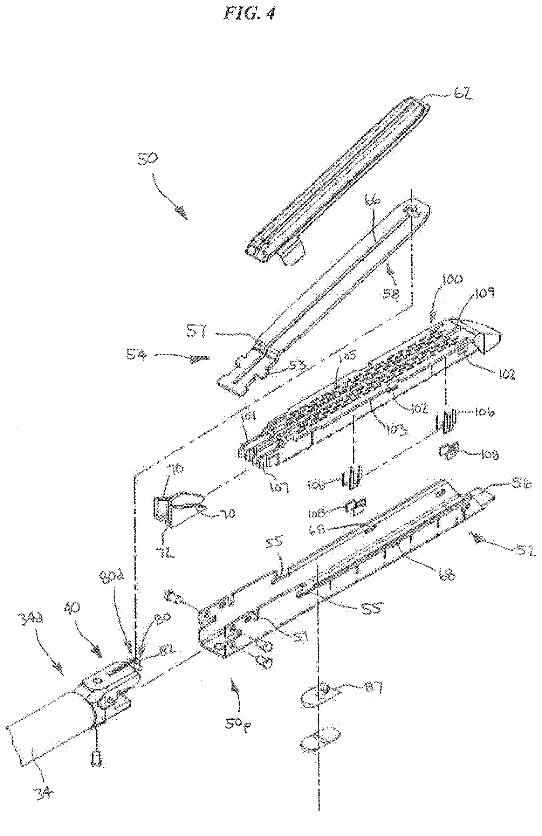

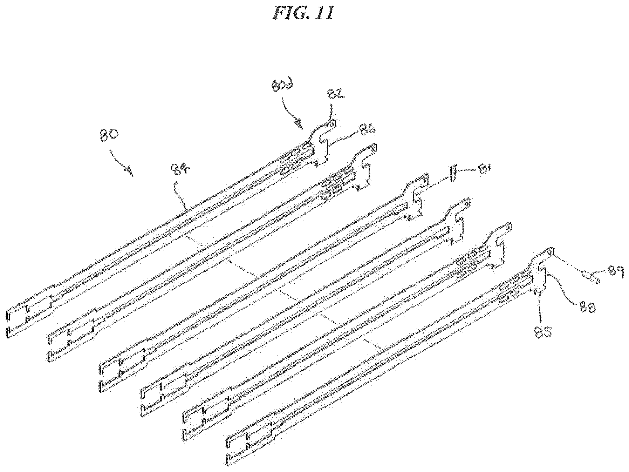

While a variety of surgical instruments can be used in conjunction with the adjunct materials disclosed herein, FIGS. 1 and 2 illustrate one, non-limiting exemplary embodiment of a surgical stapler 10 suitable for use with one or more adjunct materials. As shown the instrument 10 includes a handle assembly 12, a shaft 14 extending distally from a distal end 12d of the handle assembly 12, and an attachment portion 16 removably coupled to a distal end 14d of the shaft 14. Because the illustrated embodiment is a surgical stapler, a distal end 16d of the attachment portion 16 includes an end effector 50 having jaws 52, 54, although other types of end effectors can be used with the shaft 14, handle assembly 12, and components associated with the same. As shown, the surgical stapler includes opposed first and second jaws 52, 54 with the first, lower jaw 52 including an elongate channel 56 (FIG. 4) configured to support a staple cartridge 100, and the second, upper jaw 54 having an inner surface 58 (FIGS. 3, 4, and 6) that faces the lower jaw 52 and that is configured to operate as an anvil to help deploy staples of a staple cartridge. The jaws 52, 54 are configured to move relative to one another to clamp tissue or other objects disposed therebetween, and an axial drive assembly 80 (FIG. 11) can be configured to pass through at least a portion of the end effector 50 to eject the staples into the clamped tissue. In various embodiments a knife blade 81 can be associated with the axial drive assembly 80 to cut tissue during the stapling procedure.

Operation of the end effector 50 and drive assembly 80 can begin with input from a clinician at the handle assembly 12. The handle assembly 12 can have many different configurations designed to manipulate and operate the end effector associated therewith. In the illustrated embodiment, the handle assembly 12 has a pistol-grip type housing 18 with a variety of mechanical components disposed therein to operate various features of the instrument. For example, the handle assembly 12 can include mechanical components as part of a firing system actuated by a trigger 20. The trigger 20 can be biased to an open position with respect to a stationary handle 22, for instance by a torsion spring, and movement of the trigger 20 toward the stationary handle 22 can actuate the firing system to cause the axial drive assembly 80 to pass through at least a portion of the end effector 50 and eject staples from a staple cartridge disposed therein. A person skilled in the art will recognize various configurations of components for a firing system, mechanical or otherwise, that can be used to eject staples and/or cut tissue, and thus a detailed explanation of the same is unnecessary.

Other non-limiting examples of features that can be incorporated into the handle assembly 22 that affect manipulation and operation of an end effector associated therewith include a rotatable knob 24, an articulation lever 26, and retraction knobs 28. As shown, the rotatable knob 24 can be mounted on a forward end of a barrel portion 30 of the handle assembly 12 to facilitate rotation of the shaft 14 (or the attachment portion 16) with respect to the handle assembly 12 around a longitudinal axis L of the shaft 14. The actuation lever 26 can also be mounted on a forward end of the barrel portion 30, approximately adjacent to the rotatable knob 24. The lever 26 can be manipulated from side-to-side along a surface of the barrel portion 30 to facilitate reciprocal articulation of the end effector 50. One or more retraction knobs 28 can be movably positioned along the barrel portion 30 to return the drive assembly 80 to a retracted position, for example after the firing system has completed a firing stroke. As shown, the retraction knobs 28 move proximally toward a back end of the barrel portion 30 to retract components of the firing system, including the drive assembly 80.

Still other non-limiting examples of features that can be incorporated into the handle assembly 22 that affect manipulation and operation of an end effector associated therewith can include a firing lockout assembly, an anti-reverse clutch mechanism, and an emergency return button. A firing lockout assembly can be configured to prevent the firing system from being actuated at an undesirable time, such as when an end effector is not fully coupled to the instrument. An anti-reverse clutch mechanism can be configured to prevent components of the firing system from moving backwards when such backwards movement is undesirable, such as when the firing stroke has only been partially completed but temporarily stopped. An emergency return button can be configured to permit components of a firing system to be retracted before a firing stroke is completed, for instance in a case where completing the firing stroke may cause tissue to be undesirably cut. Although features such as a firing lockout assembly, an anti-reverse clutch mechanism, and an emergency return button are not explicitly illustrated in the instrument 10, a person skilled in the art will recognize a variety of configurations for each feature that can be incorporated into a handle assembly and/or other portions of a surgical stapler without departing from the present disclosure.

The shaft 14 can be removably coupled to the distal end 12d of the handle assembly 12 at a proximal end 14p of the shaft 14, and a distal end 14d of the shaft 14 can be configured to receive the attachment portion 16. As shown, the shaft 14 is generally cylindrical and elongate, although any number of shapes and configurations can be used for the shaft, depending, at least in part, on the configurations of the other instrument components with which it is used and the type of procedure in which the instrument is used. For example, in some embodiments, a distal end of one shaft can have a particular configuration for receiving certain types of end effectors, while a distal end of another shaft can have a different configuration for receiving certain other types of end effectors. Components of the firing system, such as a control rod 32 (FIG. 2), can be disposed in the shaft 14 so that the components can reach the end effector 50 and drive assembly 80 to provide actuation of the same. For example, when the trigger 20 operates the firing system, the control rod 32 can be advanced distally through at least a portion of the shaft 14 to cause the jaws 52, 54 to collapse towards each other and/or to drive the drive assembly 80 distally through at least a portion of the end effector 50.

The shaft 14 can also include one or more sensors (not shown) and related components, such as electronic components to help operate and use the sensors (not shown). The sensors and related components can be configured to communicate to a clinician the type of end effector associated with the distal end 14d of the shaft 14, among other parameters. Likewise, the handle assembly 12 can include one or more sensors and related components configured to communicate to a clinician the type of end effector and/or shaft associated with the distal end 12d of the handle assembly 12. Accordingly, because a variety of shafts can be interchangeably coupled with the handle assembly 12 and a variety of end effectors having different configurations can be interchangeably coupled with various shafts, the sensors can help a clinician know which shaft and end effector are being used. Additionally, the information from the sensors can help a monitoring or control system associated with the instrument know which operation and measurement parameters are relevant to a clinician based on the type of shaft and end effector coupled to the handle assembly. For example, when the end effector is a stapler, information about the number of times the drive assembly 80 is fired may be relevant, and when the end effector is another type of end effector, such as a cutting device, the distance the cutting portion traveled may be relevant. The system can convey the appropriate information to the clinician based on the end effector that is sensed.

A person skilled in the art will recognize that various configurations of monitoring and control systems can be used in conjunction with the surgical instruments provided herein. For example, sensors associated with any of the end effector 50, the attachment portion 16, the shaft 14, and the handle assembly 12 can be configured to monitor other system parameters, and a monitoring or control system can communicate to a clinician the relevant other parameters based on the type of shaft or attachment portion associated with the handle assembly.

As shown in FIG. 3, the attachment portion 16 can include a proximal housing portion 34 at a proximal end 16p thereof and an end effector or tool 50 at a distal end 16d thereof. In the illustrated embodiment, the proximal housing portion 34 includes on a proximal end 34p thereof engagement nubs 36 for releasably engaging the shaft 14. The nubs 36 form a bayonet type coupling with the distal end 14d of the shaft 14. Besides nubs 36, any number of other complementary mating features can be used to allow the attachment portion 16 to be removably coupled to the shaft 14.

A distal end 34d of the proximal housing portion 34 can include a mounting assembly 40 pivotally secured thereto. As shown in FIG. 4, the mounting assembly 40 can be configured to receive a proximal end 50p of the end effector 50 such that pivotal movement of the mounting assembly 40 about an axis perpendicular to the longitudinal axis of the housing portion 34 effects articulation of the end effector 50 about a pivot member or pin 42. This pivotal movement can be controlled by the actuation lever 26 of the handle assembly 28, with components being disposed between the lever 26 and the mounting assembly 40 to allow for movement of the lever 26 to articulate the mounting assembly 40, and thereby the end effector 50. Similar to the firing system of the instrument 10, a person skilled in the art will recognize various configurations of components for effecting articulation, mechanical or otherwise, and thus a detailed explanation of the same is unnecessary.

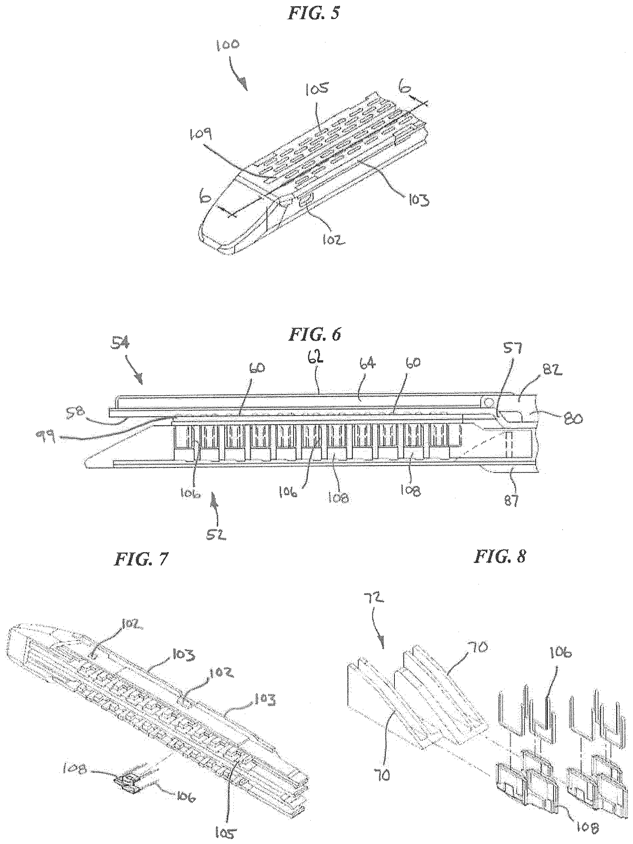

The end effector 50 of the illustrated embodiment is a surgical stapling tool having a first, lower jaw 52 that serves as a cartridge assembly or carrier and an opposed second, upper jaw 54 that serves as an anvil. As shown in FIG. 6, an inner surface 58 of the second jaw 54, sometimes referred to as an anvil portion, can include a plurality of staple deforming cavities 60 and a cover plate 62 secured to a top surface 59 of the jaw 54 to define a cavity 64 therebetween. The cover plate 62 can help to prevent pinching of tissue during clamping and firing of the surgical stapler. The cavity 64 can be dimensioned to receive a distal end 80d of the axial drive assembly 80. A longitudinal slot 66 can extend through the anvil portion 58 to facilitate passage of a retention flange 82 of the axial drive assembly 80 into the anvil cavity 64. A camming surface 57 formed on the anvil portion 58 can be positioned to engage the axial drive assembly 80 to facilitate clamping of tissue 99. A pair of pivot members 53 formed on the anvil portion 54 can be positioned within slots 51 formed in the carrier 52 to guide the anvil portion between the open and clamped positions. A pair of stabilizing members can engage a respective shoulder 55 formed on the carrier 52 to prevent the anvil portion 54 from sliding axially relative to the staple cartridge 100 as the camming surface 57 is deformed. In other embodiments, the carrier 52 and staple cartridge 100 can be pivoted between open and clamped positions while the anvil portion 54 remains substantially stationary.

The elongated support channel 56 of the first jaw 52 can be dimensioned and configured to receive a staple cartridge 100, as shown in FIGS. 4, 5, and 7. Corresponding tabs 102 and slots 68 formed along the staple cartridge 100 and the elongated support channel 56, respectively, function to retain the staple cartridge 100 within the support channel 56. A pair of support struts 103 formed on the staple cartridge 100 can be positioned to rest on sidewalls of the carrier 52 to further stabilize the staple cartridge 100 within the support channel 56. The staple cartridge 100 can also include retention slots 105 for receiving a plurality of fasteners 106 and pushers 108. A plurality of spaced apart longitudinal slots 107 can extend through the staple cartridge 100 to accommodate upstanding cam wedges 70 of an actuation sled 72 of a firing system (FIGS. 4 and 8). A central longitudinal slot 109 can extend along the length of the staple cartridge 100 to facilitate passage of a knife blade 81 associated with the axial drive assembly 80. During operation of the surgical stapler, the actuation sled 72 translates through longitudinal slots 107 of the staple cartridge 100 to advance cam wedges 70 into sequential contact with pushers 108, thereby causing the pushers 108 to translate vertically within the retention slots 105 and urge the fasteners 106 from the slots 105 into the staple deforming cavities 60 of the anvil portion 54.

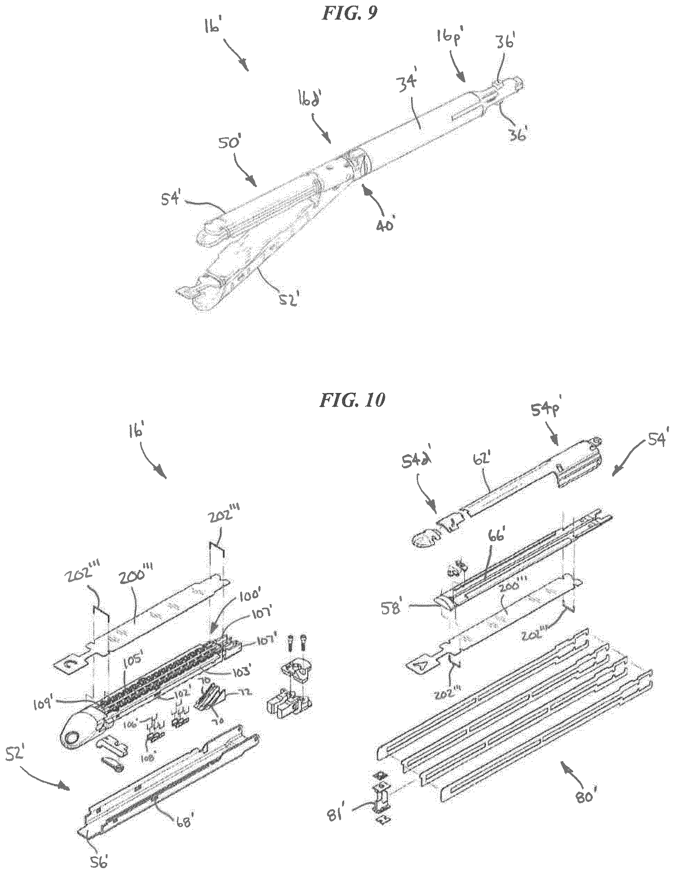

An alternative embodiment of an attachment portion 16' is shown in FIGS. 9 and 10. The attachment portion 16' can include a proximal housing portion 34' at a proximal end 16p' thereof and an end effector or tool 50' at a distal end 16d' thereof. Nubs 36' can be provided to removably couple the attachment portion 16' to a shaft of a surgical instrument, and a mounting assembly 40' can be provided to removably and/or pivotally couple an end effector or tool 50' to the proximal housing portion 34'. The end effector 50' can include a first, lower jaw 52' that serves as a cartridge assembly, and a second, upper jaw 54' that serves as an anvil portion. The first jaw 52' can have many of the same features as the first jaw 52 of FIGS. 3, 4, and 6, and thus can include an elongated support channel 56' that is dimensioned and configured to receive a staple cartridge 100', and slots 68' configured to correspond with tabs 102' of the staple cartridge 100' to retain the cartridge 100' within the channel 56'. Likewise, the cartridge 100' can include support struts 103' to rest on sidewalls of the jaw 52', retention slots 105' for receiving a plurality of fasteners 106' and pushers 108', a plurality of spaced apart longitudinal slots 107' to accommodate upstanding cam wedges 70' of an actuation sled 72' of a firing system, and a central longitudinal slot 109' to facilitate passage of a knife blade 81' associated with an axial drive assembly 80'.

Similar to the second jaw 54 of FIGS. 3, 4, and 6, the second jaw 54' can include a cover plate 62' secured to a top surface of the jaws to define a cavity therebetween. An anvil plate 58' can serve as the inner surface of the jaw 54', and can include a longitudinal slot 66' for receiving a distal end of the axial drive assembly 80', and a plurality of staple deforming pockets or cavities (not shown) to form staples ejected from the cartridge 100'. In this embodiment, however, the lower jaw 52' containing the cartridge 100' is configured to pivot toward the upper jaw 54' while the upper jaw 54' remains substantially stationary upon actuation by a handle assembly and related components.

The end effector and staple cartridge disposed therein is configured to receive an axial drive assembly. One non-limiting exemplary embodiment of the axial drive assembly 80 is illustrated in FIG. 11. As shown, a distal end of a drive beam 84 can be defined by a vertical support strut 86 that supports the knife blade 81, and an abutment surface 88 configured to engage the central portion of the actuation sled 72 during a stapling procedure. Bottom surface 85 at the base of the abutment surface 88 can be configured to receive a support member 87 slidably positioned along the bottom of the staple cartridge 100 (FIGS. 4 and 6). The knife blade 81 can be positioned to translate slightly behind the actuation sled 72 through the central longitudinal slot 109 in the staple cartridge 100 to form an incision between rows of stapled body tissue. The retention flange 82 can project distally from the vertical strut 86 and can support a cylindrical cam roller 89 at its distal end. The cam roller 89 can be dimensioned and configured to engage the camming surface 57 on the anvil portion 58 to clamp the anvil portion 58 against body tissue. A person skilled in the art will recognize that a drive assembly for use in conjunction with surgical staplers or other surgical instruments can have many other configurations than the one illustrated in FIG. 11. By way of non-limiting example, the drive assembly 80 can include a single drive beam, or any other number of drive beams, and the distal end of the drive beam(s) can have any number of shapes that are configured for use in the end effector through which the drive assembly is configured to travel.

In use, the surgical stapler can be disposed in a cannula or port and disposed at a surgical site. A tissue to be cut and stapled can be placed between the jaws 52, 54 of the surgical stapler 10. Features of the stapler 10, such as the rotating knob 24 and the actuation lever 26, can be maneuvered as desired by the clinician to achieve a desired location of the jaws 52, 54 at the surgical site and the tissue with respect to the jaws 52, 54. After appropriate positioning has been achieved, the trigger 20 can be pulled toward the stationary handle 22 to actuate the firing system. The trigger 20 can cause components of the firing system to operate such that the control rod 32 advances distally through at least a portion of the shaft 14 to cause at least one of the jaws 52, 54 to collapse towards the other to clamp the tissue disposed therebetween and/or to drive the drive assembly 80 distally through at least a portion of the end effector 50.

In some embodiments, a first firing of the trigger 20 can cause the jaws 52, 54 to clamp the tissue, while subsequent firings of the trigger 20 can cause the drive assembly 80 to be advanced distally through at least a portion of the end effector 50. A single, subsequent firing can fully advance the drive assembly 80 through the staple cartridge 100 to eject the staples in the row, or alternatively, the components in the handle assembly 12 can be configured such that multiple, subsequent firings are required to fully advance the drive assembly 80 through the staple cartridge 100 to eject the staples in the row. Any number of subsequent firings can be required, but in some exemplary embodiments anywhere from two to five firings can fully advance the drive assembly 80 through the staple cartridge 100. In embodiments in which the drive assembly 80 includes the knife 81 to cut the tissue being stapled, the knife 81 cuts tissue as the drive assembly advances distally through the end effector 50, and thus the staple cartridge 100 disposed therein. In other exemplary embodiments, a motor disposed within the handle assembly 12 and associated with a firing trigger can actuate the drive assembly 80 automatically in response to activation of the firing trigger.

After the drive assembly 80 has been advanced distally through the staple cartridge 100, the retraction knobs 28 can be advanced proximally to retract the drive assembly 80 back towards its initial position. In some configurations, the retraction knobs 28 can be used to retract the drive assembly 80 prior to fully advancing the assembly 80 through the cartridge 100. In other embodiments retraction of the drive assembly 80 can be automated to occur after a predetermined action. For example, once the drive assembly 80 has distally advanced to its desired location, the subsequent return of the trigger 80 back to a biased open position can cause the drive assembly 80 to automatically retract. A motor and associated components, rather than retraction knobs 28 and associated components, can be used to retract the drive assembly 80. Further, as discussed above, other features, such as a firing lockout mechanism, an anti-reverse clutch mechanism, and an emergency return button, can be relied upon during operation of the surgical stapler 10, as would be understood by those skilled in the art.

The illustrated embodiment of a surgical stapling instrument 10 provides one of many different configurations, and associated methods of use, that can be used in conjunction with the disclosures provided herein. Additional exemplary embodiments of surgical staplers, components thereof, and their related methods of use, that can be used in accordance with the present disclosure include those devices, components, and methods provided for in U.S. Patent Application Publication No. 2012/0083835 and U.S. Patent Application Publication No. 2013/0161374.

### IMPLANTABLE MATERIALS

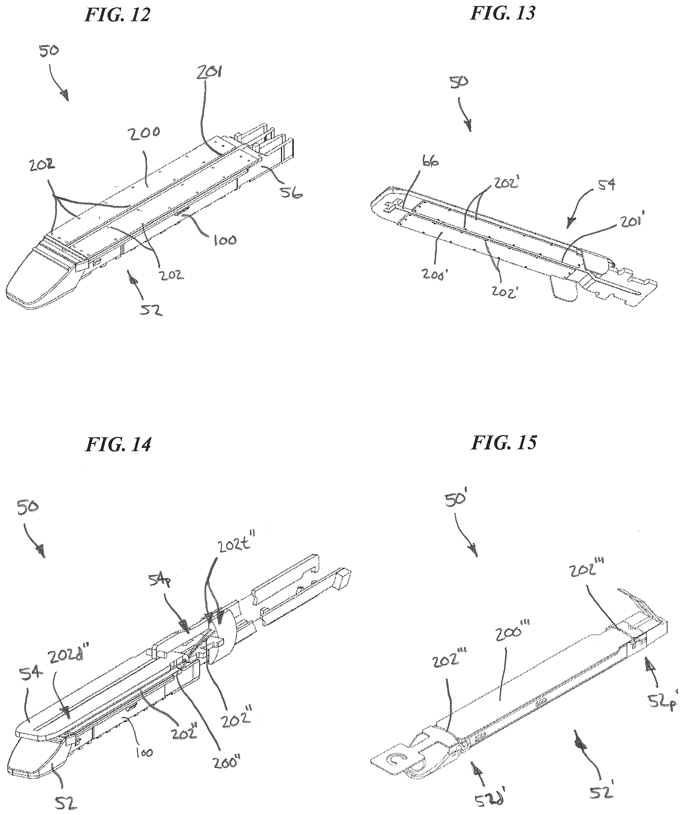

Regardless of the configuration of the surgical instrument, the present disclosure provides for the use of implantable materials, e.g., biologic materials and/or synthetic materials, collectively "adjunct materials," in conjunction with instrument operations. As shown in FIGS. 12 and 13, the end effector 50 can include at least one piece of adjunct material 200, 200' positioned intermediate the first and second jaw members 52, 54 and it can be releasably retained to one of the support channel 56 and/or the anvil portion 58. In the illustrated embodiment, the releasable retention is provided by retention members 202, 202', which are described in further detail below.

In at least one embodiment, a surface on the adjunct material 200, 200' can be configured to contact tissue as the tissue is clamped between the first and second jaw members 52, 54. In such an embodiment, the adjunct material can be used to distribute the compressive clamping force over the tissue, remove excess fluid from the tissue, and/or improve the purchase of the staples. In various embodiments, one or more pieces of adjunct material can be positioned within the end effector 50. In at least one embodiment, one piece of adjunct material 200 can be attached to the staple cartridge 100 (FIG. 12) and one piece of adjunct material 200' can be attached to the anvil portion 58 (FIG. 13). In at least one other embodiment, two pieces of adjunct material 200 can be positioned on the support channel 56 and one piece of adjunct material 200' can be positioned on the anvil portion 58, for example. Any suitable number of adjunct materials can be situated within the end effector 50.

Adjunct material used in conjunction with the disclosures provided for herein can have any number of configurations and properties. Generally, they can be formed from of a bioabsorbable material, a biofragmentable material, and/or a material otherwise capable of being broken down, for example, such that the adjunct material can be absorbed, fragmented, and/or broken down during the healing process. In at least one embodiment, the adjunct material can include a therapeutic drug that can be configured to be released over time to aid the tissue in healing, for example. In further various embodiments, the adjunct materials can include a non-absorbable and/or a material not capable of being broken down, for example. Similarly, the connection or retention members can be at least partially formed from at least one of a bioabsorbable material, a biofragmentable material, and a material capable of being broken down such that the retention members can be absorbed, fragmented, and/or broken down within the body. In various embodiments, the retention members can include a therapeutic drug that can be configured to be released over time to aid the tissue in healing, for example. In further various embodiments, the retention members can include a non-absorbable and/or a material not capable of being broken down, for example, such as a plastic.

More particularly, some exemplary, non-limiting examples of synthetic materials that can be used in conjunction with the disclosures provided for herein include biodegradable synthetic absorbable polymer such as a polydioxanon film sold under the trademark PDS^{®} or with a Polyglycerol sebacate (PGS) film or other biodegradable films formed from PGA (Polyglycolic acid, marketed under the trade mark Vicryl), PCL (Polycaprolactone), PLA or PLLA (Polylactic acid), PHA (polyhydroxyalkanoate), PGCL (poliglecaprone 25, sold under the trademark Monocryl), PANACRYL (Ethicon, Inc., Somerville, N.J.), Polyglactin910, Poly glyconate, PGA/TMC (polyglycolide-trimethylene carbonate sold under the trademark Biosyn), polyhydroxybutyrate (PHB), poly(vinylpyrrolidone) (PVP), poly(vinyl alcohol) (PVA), or a blend of copolymerization of the PGA, PCL, PLA, PDS monomers. In use, the synthetic material can be broken down by exposure to water such that the water attacks the linkage of a polymer of the synthetic material. As a result, the mechanical strength can become diminished, and a construct of the material can be broken down into a mushy or fractured scaffold. As further breakdown occurs such that the material breaks into carbohydrates and acid constituents, a patient's body can metabolize and expel the broken down materials.

Some exemplary, non-limiting examples of biologic derived materials that can be used in conjunction with the disclosures provided for herein include platelet poor plasma (PPP), platelet rich plasma (PRP), starch, chitosan, alginate, fibrin, thrombin, polysaccharide, cellulose, collagen, bovine collagen, bovine pericardium, gelatin-resorcin-formalin adhesive, oxidized cellulose, mussel-based adhesive, poly (amino acid), agarose, polyetheretherketones, amylose, hyaluronan, hyaluronic acid, whey protein, cellulose gum, starch, gelatin, silk, or other material suitable to be mixed with biological material and introduced to a wound or defect site, including combinations of materials, or any material apparent to those skilled in the art in view of the disclosures provided for herein. Biologic materials can be derived from a number of sources, including from the patient in which the biologic material is to be implanted, a person that is not the patient in which the biologic material is to be implanted, or other animals.

Additional disclosures pertaining to synthetic or polymer materials and biologic materials that can be used in conjunction with the disclosures provided herein can be found in U.S. Patent Application Publication No. 2012/0080335, U.S. Patent Application Publication No. 2012/0083835, U.S. Patent Application Serial No. 13/433,115, entitled "Tissue Thickness Compensator Comprising Capsules Defining a Low Pressure Environment," and filed on March 28, 2012, U.S. Patent Application Serial No. 13/433,118, entitled "Tissue Thickness Compensator Comprised of a Plurality of Materials," and filed on March 28, 2012, U.S. Patent Application Serial No. 13/532,825, entitled "Tissue Thickness Compensator Having Improved Visibility," and filed on June 26, 2012, U.S. Patent Application Serial No. 13/710,931, entitled "Electrosurgical End Effector with Tissue Tacking Features," and filed on December 11, 2012, and U.S. Patent Application Serial No. 13/763,192, entitled "Multiple Thickness Implantable Layers for Surgical Stapling Devices," and filed on February 8, 2013.

In use, the adjunct material can come pre-loaded onto the device and/or the staple cartridge, while in other instances the adjunct material can be packaged separately. In instances in which the adjunct material comes pre-loaded onto the device and/or the staple cartridge, the stapling procedure can be carried out as known to those skilled in the art. For example, in some instances the firing of the device can be enough to disassociate the adjunct material from the device and/or the staple cartridge, thereby requiring no further action by the clinician. In other instances any remaining connection or retention member associating the adjunct material with the device and/or the staple cartridge can be removed prior to removing the instrument from the surgical site, thereby leaving the adjunct material at the surgical site. In instances in which the adjunct material is packaged separately, the material can be releasably coupled to at least one of a component of the end effector and the staple cartridge prior to firing the device. The adjunct material may be refrigerated, and thus removed from the refrigerator and the related packaging, and then coupled to the device using a connection or retention member as described herein or otherwise known to those skilled in the art. The stapling procedure can then be carried out as known to those skilled in the art, and if necessary, the adjunct material can be disassociated with the device as described above.

### RETENTION MEMBERS

Connection or retention members can be used to secure, at least temporarily, one or more pieces of adjunct material onto an end effector and/or staple cartridge. These retention members can come in a variety of forms and configurations, such as one or more sutures, adhesive materials, staples, brackets, snap-on or other coupling or mating elements, etc. For example, the retention members can be positioned proximate to one or more sides and/or ends of the adjunct material, which can help prevent the adjunct material from peeling away from the staple cartridge and/or the anvil face when the end effector is inserted through a trocar or engaged with tissue. In still other embodiments, the retention members can be used with or in the form of an adhesive suitable to releasably retain the adjunct material to the end effector, such as cyanoacrylate. In at least one embodiment, the adhesive can be applied to the retention members prior to the retention members being engaged with the adjunct material, staple cartridge, and/or anvil portion. Generally, once firing is completed, the retention member(s) can be detached from the adjunct material and/or the end effector so that the adjunct material can stay at the surgical site when the end effector is removed. Some exemplary, non-limiting embodiments of retention members are described herein with respect to FIGS. 12-15.

FIG. 12 illustrates one exemplary embodiment of a connection or retention member 202 associated with the adjunct material 200 to secure the material 200 at a temporary location with respect to the lower jaw 52 of the end effector 50. As shown, the adjunct material 200 is disposed over the staple cartridge 100 located in the elongate channel 56 of the lower jaw 52, and the retention member 202 extends therethrough. In the embodiment, the retention member 202 is in the form of a single suture stitched through multiple locations of the adjunct material 200, or it can be multiple sutures disposed at one or more locations on the adjunct material 200. As shown, the sutures are positioned at locations around a perimeter of the adjunct material 200, and are also adjacent to a central longitudinal channel 201 formed in the adjunct material 200. The channel 201 can make it easier for a knife passing through the adjunct material 200 to cut the material 200 into two or more separate strips. In some embodiments, for instance when the retention member 202 is a single suture threaded through multiple locations of the adjunct material 200, a knife passing through the lower jaw 52 can cut the retention member 202 at one or more locations, thereby allowing the retention member 202 to be disassociated from the adjunct material 200 and removed from the surgical site while the adjunct material 200 remains held at the surgical site by one or more staples ejected from the cartridge 100.

FIG. 13 illustrates another embodiment of a connection or retention member 202' associated with the adjunct material 200' to secure the material 200' at a temporary location on the end effector 50. The retention member 202' has the same configuration as the retention member 202 in FIG. 12, however, in this embodiment it is used to secure the material to the anvil or upper jaw 54, rather than the cartridge or lower jaw 52.

FIG. 14 illustrates another, non-limiting embodiment of a connection or retention member 202" used to releasably retain an adjunct material 200" to at least one of the upper jaw 54 and the lower jaw 52. In this embodiment, the retention member 202" is a single suture that extends through a distal portion 200d" of the adjunct material 200" and is coupled to a proximal end 54p of the upper jaw 54. Terminal ends 202t" of the retention member 202" can be used to move the retention member 202" with respect to the jaws 54, 52. In its extended position, which is illustrated in FIG. 14, the retention member 202" can hold the adjunct material 200" in position as the end effector 50 is inserted into a surgical site. Thereafter, the jaws 52, 54 of the end effector 50 can be closed onto tissue, for example, and staples from the staple cartridge 100 can be deployed through the adjunct material 200" and into the tissue. The retention member 202" can be moved into its retracted position such that the retention member 202" can be operably disengaged from the adjunct material 200". Alternatively, the retention member 202" can be retracted prior to the staples being deployed. In any event, as a result of the above, the end effector 50 can be opened and withdrawn from the surgical site leaving behind the adjunct material 200" and tissue.

FIG. 15 illustrates yet another, non-limiting embodiment of a connection or retention member 202''' for securing a location of adjunct material 200'" to an end effector. In particular, the adjunct material 200'" and retention member 202'" are used in conjunction with the end effector 50' of FIGS. 9 and 10. In this embodiment, the retention member 202'" is in the form of a suture that is used to tie the adjunct material 200'" to the first, lower jaw 52' at proximal and distal ends thereof 52p', 52d'. Similarly, as shown in FIGS. 9 and 10, the adjunct material 200'" can also be secured to the second, upper jaw 54' at proximal and distal ends thereof 54p', 54d'. Optionally, recesses can be formed in either or both of the jaws 52', 54', and either or both of the adjunct materials 200"', which can protect the retention members 202'" against unintended cutting by an outside object. In use, the knife blade 81' on the driver assembly 80' can incise the retention members 202'" as it passes through the end effector 50' to release the adjunct material 200"'.

A person skilled in the art will recognize a variety of other ways by which the adjunct material can be temporarily retained with respect to the end effector. In various embodiments a connection or retention member can be configured to be released from an end effector and deployed along with a piece of adjunct material. In at least one embodiment, head portions of retention members can be configured to be separated from body portions of retention members such that the head portions can be deployed with the adjunct material while the body portions remain attached to the end effector. In other various embodiments, the entirety of the retention members can remain engaged with the end effector when the adjunct material is detached from the end effector.

### HYBRID ADJUNCT MATERIAL

The retention members provided for herein, or otherwise known to those skilled in the art, can be used in conjunction with a variety of adjunct materials. While in some instances the adjunct materials can be either a synthetic material or a biologic material, in some exemplary embodiments the adjunct material can include both synthetic material(s) and biologic material(s), referred to herein as a hybrid adjunct material. The resulting combination can advantageously have both permeable and non-permeable elements, and allows for the beneficial features of both types of adjunct materials to be incorporated into a single adjunct material. For example, synthetic material can provide structure and support for biologic material, and can add strength and shear resistance to fibrous biologic material, while still being configured to allow the biologic material to have direct access to a surgical site so the biologic material can provide improved healing and tissue growth at the stapled location. Depending on the type of material that is used, either or both synthetic and biologic material can help seal holes formed by staples as they are implanted into tissue, blood vessels, and various other objects or body parts. Further, either or both of the synthetic and biologic materials can be configured to help reduce inflammation, promote cell growth, and otherwise improve healing.

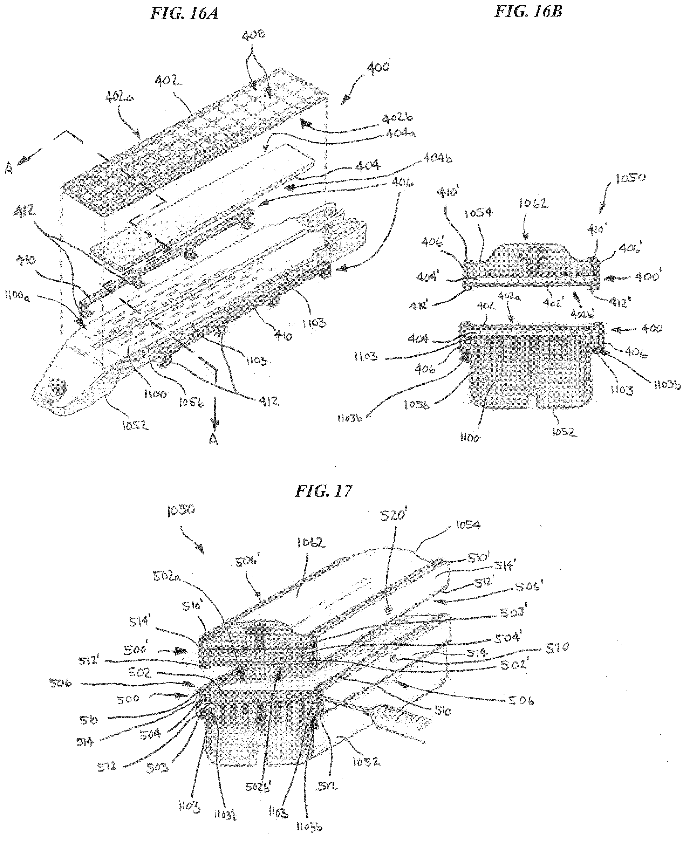

A hybrid adjunct material can be selectively attached to either or both jaws of an end effector. As shown in FIGS. 16A and 16B, a hybrid adjunct material 400, 400' is attached to both lower and upper jaws 1052, 1054 of an end effector 1050. The hybrid adjunct material 400, 400' of the illustrated embodiment includes a synthetic material, layer, or matrix 402, 402' in the form of a polymer mesh, and a biologic material, layer, or matrix 404, 404' in the form of a bioabsorbable membrane. The terms material, layer, and matrix are often used interchangeably herein, and to the extent any of these terms are used, the terms are not so limiting as to require a particular shape, thickness, or configuration. A person skilled in the art will recognize a variety of configurations that the synthetic and biologic materials can have that allow them to be used in conjunction with an end effector without departing from the present disclosure. As described herein, the synthetic and biologic layers 402, 402' and 404, 404' can be coupled to the lower and upper jaws 1052, 1054 using a variety of techniques, but in the illustrated embodiment a pair of brackets 406 is used to maintain a location of the hybrid adjunct material 400 with respect to the cartridge assembly or lower jaw 1052. Likewise, a pair of brackets 406' is used to maintain a location of the hybrid adjunct material 400' with respect to the upper jaw 1054.

The lower jaw 1052 and associated hybrid adjunct material 400 are illustrated in FIG. 16A. Similar to some of the embodiments described herein, the lower jaw 1052 can have a staple cartridge 1100 disposed therein. The staple cartridge 1100 can include staples for deployment at the surgical site, and as shown, can include support struts 1103. The support struts 1103 can help stabilize the cartridge 1100 within a support channel 1056 of the lower jaw 1052, and can also be engaged by the brackets 406 to help temporarily secure the hybrid adjunct material 400 to the lower jaw 1052.

The biologic layer 404 can have many different configurations in terms of its size, shape, and the materials of which it is comprised, but in the illustrated embodiment the biologic layer 404 is substantially planar and rectangular, and includes a bioabsorbable membrane. The biologic layer 404 can be in the form of an extracellular matrix, and/or it can include patient-derived materials such as platelet enriched plasma, diced tissue fragments, fibrin, and stem cells. Other types of biologic materials that can be incorporated into the biologic layer 404 are provided earlier in this disclosure. As shown in FIGS. 16A and 16B, a bottom surface 404b of the biologic layer 404 is facially opposed to a top surface 1100a of the staple cartridge 1100 and the two surfaces 404b, 1100a are in contact with each other.

The synthetic layer 402 can likewise have many different configurations in terms of its size, shape, and the materials of which it is comprised. In the illustrated embodiment the synthetic layer 402 is substantially planer and rectangular and includes a plurality of openings 408 formed therein to provide a lattice structure or mesh. This open configuration allows components of the biologic layer 404 to pass through the synthetic layer 402 and provide desired healing to tissue at the surgical site, while still providing a support structure for the biologic layer 404. If the synthetic layer 402 was not permeable and contained no openings, it could act as a barrier between the tissue and the biologic layer 404. Thus, in instances in which there are no openings, typically the synthetic layer 402 is permeable. Any number of materials can be used to form the synthetic layer 402, including those described above, but in some embodiments a polymer is used. Further, healing agents and/or biologic materials can be incorporated into the synthetic layer 402, for instance by painting a layer of the agents and/or biologic materials on a top surface 402a of the synthetic layer 402. As shown in FIGS. 16A and 16B, a bottom surface 402b of the synthetic layer 402 is facially opposed to a top surface 404a of the biologic layer 404 and the two surfaces 402b, 404a are in contact with each other.

The one or more brackets 406 can be used to maintain the location of the biologic and synthetic layers 404, 402 with respect to the staple cartridge 1100 and lower jaw 1052. In the illustrated embodiment, two opposed brackets 406 are configured to engage a bottom surface 1103b of the strut 1103 and the top surface 402a of the synthetic layer 402 to maintain the location of the layers 404, 402. The brackets 406 can have any number of shapes, sizes, and configurations, but in the illustrated embodiment of FIGS. 16A and 16B, a channel 410 for engaging the top surface 402a of the synthetic layer 402 extends the length of a top portion 406a of the bracket 406, and a plurality of engagement tabs 412 for engaging the bottom surface 1103b of the struts 1103 extend from the channel 410. As also shown in FIG. 16B, the hybrid adjunct material 400' that includes the biologic layer 404' and the synthetic layer 402' can also be associated with an anvil or upper jaw 1054 of the end effector 1050, for instance by using opposed brackets 406'. As shown, top channels 410' of the brackets 406' engage a surface of a cover plate 1062 of the upper jaw 1054, and the bottom tabs 412' engage a bottom surface 402b' of the synthetic layer 402'. The synthetic and biologic layers 402', 404' associated with the upper jaw 1054 can have the same size, shape, and composition as the synthetic and biologic layers 402, 404 associated with the lower jaw 1052, or the sizes, shapes, and compositions can be different. Additional details about associating a hybrid adjunct material with an anvil are provided further below.

FIG. 17 provides another configuration of hybrid adjunct materials 500, 500' associated with each of the lower and upper jaws 1052, 1054. As shown, a biologic layer 504 associated with the lower jaw 1052 is disposed between two synthetic layers 502, 503, and a biologic layer 504' associated with the upper jaw 1054 is disposed between two synthetic layers 502', 503'. The synthetic layers 502, 502' can be permeable so as to allow biologic materials from the respective biologic layers 504, 504' to pass through the synthetic layer 502, 502' and interact with surrounding tissue upon deployment. The synthetic layer 503, 503' can also be permeable. Optionally, either or both of the synthetic layers 502, 502' and 503, 503' can have one or more openings formed therein to allow biologic material to pass therethrough. In one exemplary embodiment, the synthetic layers 502, 502' and 503, 503' are an absorbable alginate membrane and the biologic layers 504, 504' include platelet rich plasma (PRP). The synthetic and biologic materials associated with the lower and upper jaws 1052, 1054 can be, but do not have to be, the same shape, size, and/or composition.

Opposed brackets 506 can be used to maintain the location of the synthetic and biologic layers 502, 503, 504 with respect to the lower jaw 1052, and opposed brackets 506' can be used to maintain the location of the synthetic and biologic layers 502', 503', 504' with respect to the upper jaw 1054. While any configuration of bracket can be used, in the illustrated embodiment the brackets 506, 506' include top and bottom channels 510, 512 and 510', 512' that extend a length of the brackets 506, 506' and an end wall 514, 514' that connects the two channels 510, 512 and 510', 512'. As shown, the bottom channel 512 engages the bottom surface 1103b of the struts 1103 and the top channel 510 engages a top surface 502a of the synthetic layer 502, while the bottom channel 512' engages a bottom surface 502b of the synthetic layer 502 and the top channel 510' engages a surface of the cover plate 1062 of the upper jaw 1054.

Optionally, one or more ports 520, 520' can be formed in the hybrid adjunct material 500, 500' and/or the brackets 506, 506' to allow materials, such as patient-derived materials, including fluids, to be injected into the hybrid adjunct material 500, 500'. The ports 520, 520' can be non-permeable and self-sealing. In the illustrated embodiment, the ports 520, 520' extend through the brackets 506, 506' and into the biologic layers 504, 504', however in other embodiments, such as those in FIGS. 16A and 16B in which the brackets 406, 406' do not cover a length-wise edge of the hybrid adjunct material 400, 400', the ports can be formed in one or both of the synthetic and biologic layers 502, 502' and 504, 504' without being formed in the brackets.

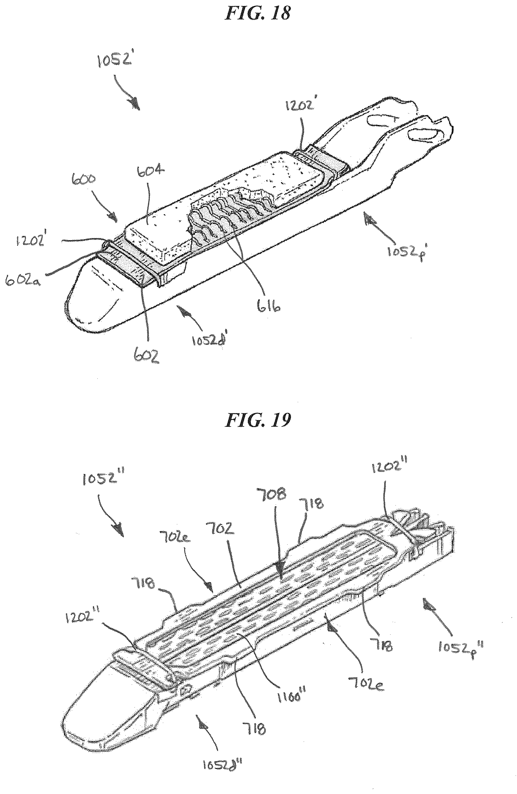

In other embodiments of a hybrid adjunct material, a synthetic layer can be coupled to a jaw of the end effector and can include one or more mating features for receiving and coupling to a biologic layer. For example, FIG. 18 illustrates a lower jaw 1052' having a hybrid adjunct material 600 associated therewith. A synthetic material, layer, or matrix 602 of the hybrid adjunct material 600 can be coupled to the jaw 1052' using retention members 1202' extending across proximal and distal ends 1052p', 1052d' thereof. Further, the synthetic matrix 602 can include one or more protrusions, as shown springs 616, which are adapted to engage a biologic material, layer, or matrix 604 to mate the biologic matrix 604 to the synthetic matrix 602, thereby substantially maintaining a location of the biologic matrix 604 with respect to the synthetic matrix 602 and the lower jaw 1052'. In the illustrated embodiment, the springs 616 form a skeletal structure around which the biologic matrix 604 can be formed. For example, when the biologic matrix 604 is formed from collagen, which as discussed elsewhere in this disclosure can be melted into its aqueous state and then reformed into a hardened state, the synthetic matrix can be dipped in the collagen when the collagen is in its aqueous state. As the collagen reforms or hardens, it can form around the skeletal structure defined by the configuration of the springs 616, thereby integrating the biologic matrix with the synthetic matrix during refinement. The resulting hybrid adjunct material can be a macro-composite adjunct that benefits from the strength and tear resistance of the internal synthetic frame and the simple parameter attachment features provided by the springs 616, *e.g*., the shape and material of the springs, while still providing for the benefits of having biologic material at the implantation site. A person skilled in the art will recognize a variety of other protrusions that can extend from a top surface 602a of the synthetic matrix 602 in any number of configurations to provide an internal skeletal structure for forming a hybrid adjunct material.

Synthetic materials or layers can have a variety of other configurations that are conducive to both providing a support structure for the biologic materials or layers while permitting the biologic materials to pass therethrough so that they can interact with tissue engaged by the staple. Various configurations are illustrated herein. As shown in FIG. 19, a synthetic material, layer, or matrix 702 is coupled to a lower jaw 1052" by retention members 1202", which as shown are sutures, disposed at proximal and distal ends 1052p", 1052d" thereof. The synthetic layer 702 can include a large, central opening 708 formed therein to permit biologic materials disposed above the synthetic layer 702 to interact with the staples disposed in a staple cartridge 1100" below, as well as the tissue in which the staples are injected. Accordingly, the synthetic layer 702 can serve as a frame for the hybrid adjunct material 700, configured to only be disposed around a perimeter of a biologic layer disposed on top of the synthetic layer 702. Shoulders 718 formed on outer edges 702e of the synthetic layer 702 can provide structure that at least one of a biologic layer and a coupling mechanism like a bracket can engage to attach the biologic layer to the synthetic layer 702, and thus the lower jaw 1052".

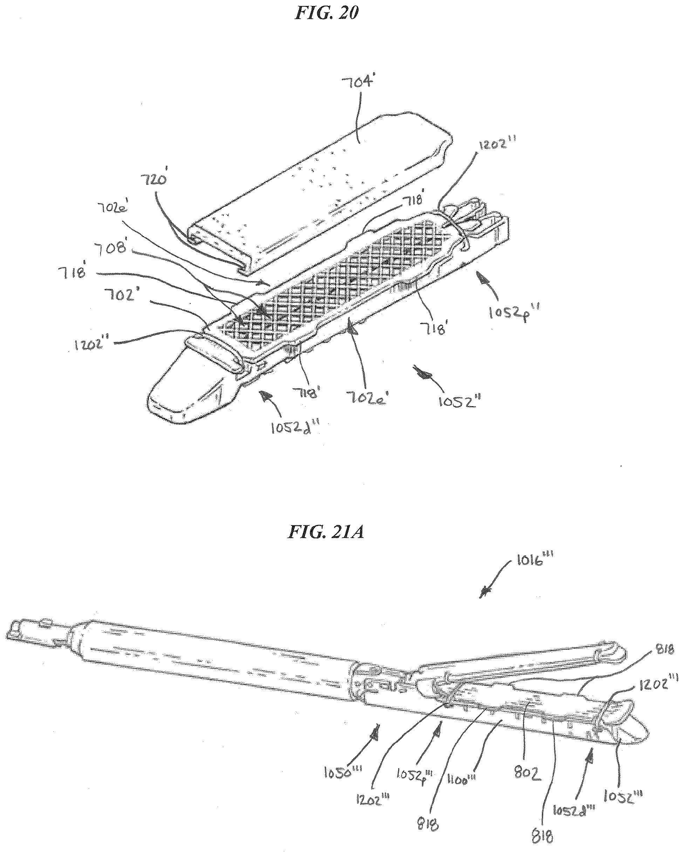

The lower jaw 1052" of FIG. 20 also provides for a synthetic material, layer, or matrix 702' that permits biologic materials to pass therethrough. As shown, the synthetic layer 702' is coupled to the lower jaw 1052" by retention members 1202", *e.g*., sutures, disposed at proximal and distal ends 1052p", 1052d" thereof. A plurality of openings 708' can be formed therein such that the synthetic layer 702' has a matrix or lattice structure, with the bars forming the lattice extending diagonally to a length of the layer 702' and substantially perpendicular with respect to each other. Similar to openings in other synthetic materials, the openings 708' can permit biologic materials to pass therethrough. The synthetic layer 702' can include shoulders or tabs 718' formed on its edges 702e'. As a result, a biologic material such as the material, layer, or matrix 704' illustrated in FIG. 20 can have channels 720' formed therein that are configured to engage the shoulders 718', as described in further detail below with respect to a related embodiment illustrated in FIGS. 21A-E.

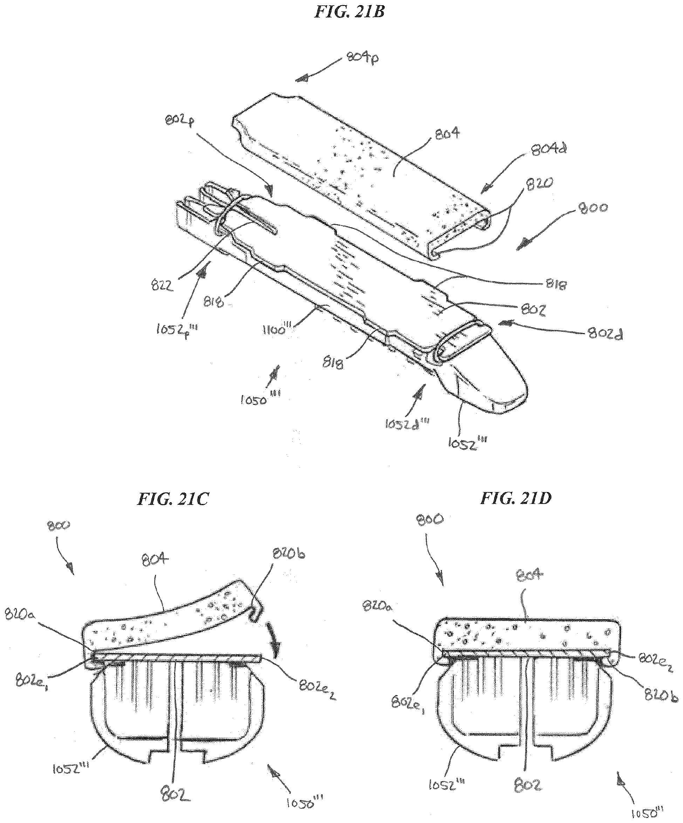

The biologic layer can be coupled to the synthetic layer using a number of techniques, such as the brackets discussed above. In another exemplary embodiment one of the synthetic and biologic layers can be configured to form a snap-fit with the other layer. As shown in FIG. 21A, a synthetic material layer 802 is disposed over a staple cartridge 1100'" and coupled to a lower jaw 1052"' of an end effector 1050'" of an attachment portion 1016'" by retention members 1202'" disposed at proximal and distal ends 1052p"', 1052d'" thereof. The synthetic layer 802 can be generally permeable, and can include shoulders 818 for receiving a biologic material, layer, or matrix 804 (FIG. 21B) to form a hybrid adjunct material 800. Further, as shown in FIG. 21B, the synthetic layer 802 can include a groove 822 formed at a proximal end 802p thereof for receiving a knife that passes through the end effector 1050"'. The groove 822 enables the knife to cut the synthetic layer 802 to release the synthetic and biologic layers 802, 804 from the end effector 1050'" and allows the layers 802, 804 to be secured at the treatment location by staples of the staple cartridge 1100"'.

The biologic layer 804 can include opposed channels 820 configured to form a snap-fit with the synthetic layer 802. As shown in FIG. 21B, the channels 820 extend along a substantial length of the biologic layer 804. A proximal end 804p of the biologic layer 804 can include a portion that is sized to be complementary with a proximal end 802p of the synthetic layer 802. Accordingly, as shown, the proximal end 804p of the biologic layer 804 can have a smaller width than a distal end 804d thereof, just as the proximal end 802p of the synthetic layer 802 also has a smaller width than a distal end 802d thereof.

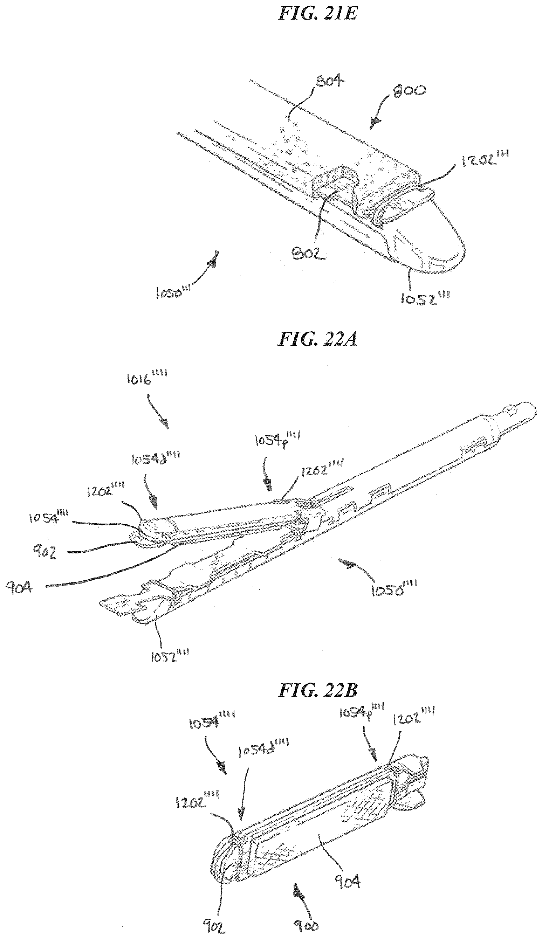

As shown in FIG. 21C, a first lengthwise outer edge 802e₁ of the synthetic layer 802 can be disposed in a first channel 820a of the biologic layer 804. The biologic layer 804, which can be pliable, can be flexed to allow a second lengthwise outer edge 802e₂ of the synthetic layer 802 to be disposed in a second channel 820b of the biologic layer 804. The resulting configuration is illustrated in FIGS. 21D and 21E. The fit between the biologic layer 804 and the synthetic layer 802 can generally be of the nature that, once coupled along both lengthwise edges, leaves the biologic layer 804 generally free of stress and tension. Further, the synthetic layer 802 can provide support for the biologic layer 804 to prevent it from easily falling apart. As discussed in greater detail below, as staples are ejected by a knife passing through the end effector 1050"', the hybrid adjunct material 800 is maintained at the surgical site by the staple, and becomes disassociated with the end effector 1050'" when the knife cuts the retention members 1202"'. Even though a matrix or other openings are not overtly formed in the illustrated synthetic layer 802, the synthetic layer 802 can be permeable, thereby allowing materials from the biologic layer 804 to pass therethrough before, during, and after staple delivery. In other embodiments the synthetic layer 802 can include a matrix, lattice, or other structure containing one or more openings as described above to permit passage of biologic materials from the biologic layer to tissue being stapled.

As illustrated in FIGS. 16B and 17, a hybrid adjunct material 400', 500' can be associated with an anvil or upper jaw 1054 in addition to or in lieu of associating the hybrid adjunct material 400, 500 with the cartridge assembly or lower jaw 1052 of the end effector 1050. In those earlier described embodiments, brackets 406, 506 and 406', 506' are used to maintain a location of the hybrid adjunct materials 400, 500 and 400', 500' with respect to the lower and upper jaws 1052 and 1054. FIGS. 22A and 22B illustrate another embodiment in which a hybrid adjunct material 900 is associated with an anvil or upper jaw 1054"" of an end effector 1050"" of an attachment portion 1016"". As shown, a synthetic material, layer, or matrix 902 is coupled to the anvil 1054"" using retention members 1202"" on proximal and distal ends 1054p"", 1054d"" of the anvil 1054"". A biologic material, layer, or matrix 904 is coupled to the anvil 1054"" in a manner similar to the manner described above with respect to the lower jaw 1052' of FIG. 18. Accordingly, one or more protrusions (not shown) can extend from the synthetic layer 902 and into the biologic layer 904 such that the two layers 902, 904 are coupled together.

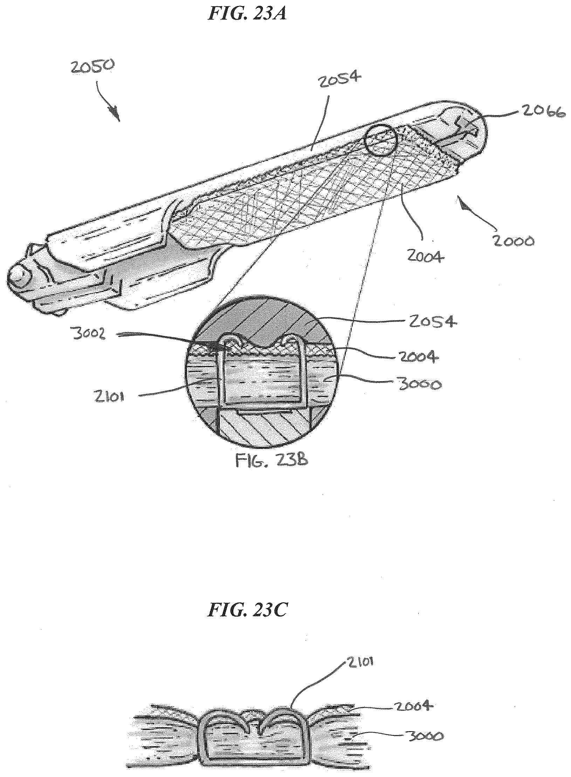

FIGS. 23A-23C provide for an embodiment of an adjunct material 2000 associated with an anvil or upper jaw 2054 that is completely biologic. Such a configuration can still be considered a hybrid adjunct material to the extent it incorporates multiple biologic materials. As shown, the biologic layer 2004 includes a collagen matrix configured to couple to the anvil 2054 of an end effector 2050. The formation of the biologic layer 2004 can be achieved by purifying and refining collagen. The collagen purification and refinement process can suspend the collagen in an aqueous state. While in this state, fats and other impurities of the collagen can be skimmed off. The aqueous collagen can then be formed into a desired biologic layer shape. For example, the aqueous collagen can be poured into a mold having inverse pockets formed therein that are complementary to the shape of an interior surface of the anvil 2054. Temperature and surface conditions of the mold can be controlled or otherwise tuned by the user to control parameters of the resulting layer, such as the density. In some embodiments an approximately uniform density can be achieved across the layer, while in other embodiments the temperature and surface conditions can be tuned such that the density of the biologic layer 2004 is not uniform across its body. For example, density variants can be formed around inverse pocket shapes formed in the biologic layer 2004. As a result, once the collagen solidifies, the biologic layer 2004 can be keyed into the anvil 2054 of the end effector 2050. In some embodiments, a protrusion (not shown) can be formed that is complementary to a longitudinal slot 2066 formed in the anvil 2054 such that protrusion on the biologic layer 2004 can help to maintain the location of the biologic layer 2044 with respect to the anvil 2054 before and during staple ejection.

In the illustrated embodiment, the biologic layer 2004 is made from biologic material that has adhesive or semi-adhesive properties. By way of non-limiting example, the biologic layer 2004 can be formed by using a thin film of polyglycolic acid (PGA)/poly (ε-caprolactone (PCL), which in a thin film acts as a semi-adhesive. In one exemplary embodiment the PGA/PCL balance is approximately 65/35, although other combinations can be used. A film having approximately this configuration can be such that after the staples are ejected, the remaining collagen would be minimal. More particularly, when compressed against tissue 3000 during clamping the main collagen body of the biologic layer 2004 and the pockets can be crushed, thus, as shown in FIG. 23B, creating a layer that would easily be penetrated by a staple 2101 but would prevent tissue from entering the staple forming area or staple pocket 3002. Still further, after the staple 2101 is fully formed, the collagen matrix of the biologic layer 2004 can be configured to swell and fill gaps, as shown in FIG. 23C. For example, liquids such as hydrogel, oxidized regenerated cellulose (ORC), or alginate can be included as part of the collagen matrix, which help to seal around the legs of the staple 2101 and minimize damage during stapling. As a result, both during and after staple firing, the potential for damage to the vessel, and the potential for bleeding at the surgical site, are reduced.

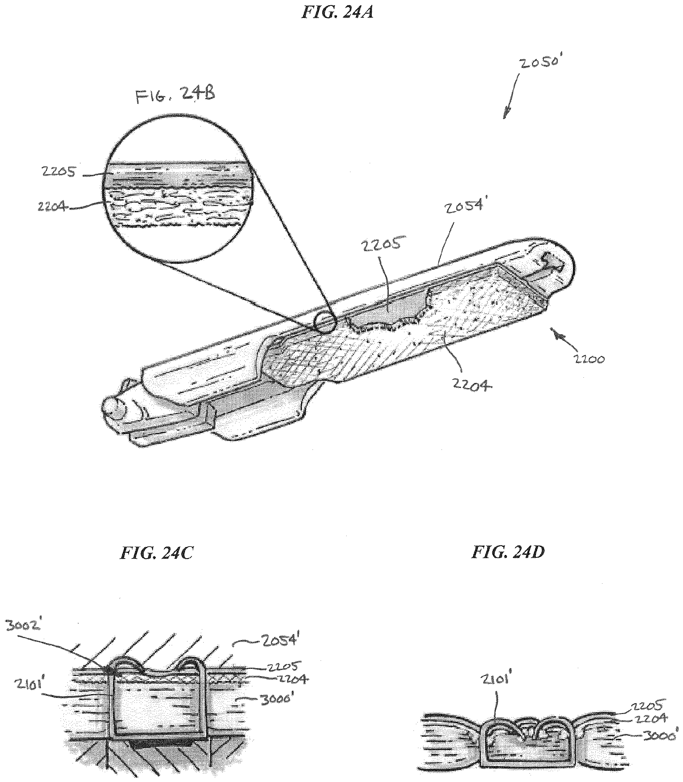

FIGS. 24A-D illustrate another embodiment of an adjunct material 2200 configured to couple to an anvil or upper jaw 2054' of an end effector 2050'. As shown the adjunct material 2200 includes two biologic layers-a first layer 2204 that includes ORC gel and a second layer 2205 that is formed, at least in part, from omentum and serves as a scaffold or support for the first layer 2204. The first layer 2204 can create a viscous layer capable of damming up bleeding, thereby allowing the body to more readily clot. In alternative embodiments, as described above, a layer of ORC gel can be used in conjunction with a thin film like PGA/PCL. The film of PGA/PCL can help prevent the ORC gel from activating too quickly when in contact with body fluids, and can help provide shear, tear, and/or axial strength.

The second layer 2205 can also provide shear, tear, and axial strength for the adjunct material 2200. Omentum is a biologically derived adjunct, and as shown can be formed into a scaffold to help support the ORC gel of the first layer 2204. Further, omentum is generally compatible with tissue, is capable of mitigating bleeding, and can generally assist in the tissue healing process. The first layer 2204 can be coupled to the second layer 2205 using any techniques known to those skilled in the art and/or described herein. For example, they can be mechanically attached similar to the embodiment of FIG. 18. Alternatively, an adhesive or semi-adhesive collagen layer can be disposed therebetween to serve as a coupling agent. Likewise, the second layer 2205 can be coupled to the anvil 2054' using any techniques known to those skilled in the art and/or described herein. In the illustrated embodiment, an adhesive material such as collagen (not shown) can be applied to either or both of the second layer 2205 and the anvil 2054' to maintain the location of the second layer 2205, and thus the first layer 2204 coupled thereto, with respect to the anvil 2054'. In some embodiments, a third layer (not shown), which like the second layer 2205 can include omentum, can be provided and the first layer 2204 can be sandwiched between the two layers that include omentum. For example, the third layer can be is disposed more proximate to a staple cartridge disposed in a cartridge assembly or lower jaw than the second layer 2205, and thus can help shield the ORC gel of the first layer 2204 from premature activation by tissue disposed between the jaws of the end effector 2050'.

As a staple 2101' is ejected from the cartridge 2100' and into tissue 3000', the first layer 2204 helps prevent tissue from entering the staple forming area or staple pocket 3002', as shown in FIG. 24C. The tissue 3000' at the surgical site can activate the ORC gel of the first layer 2204. Accordingly, the ORC gel of the first layer 2204 can begin to melt as it becomes wet. As the ORC melts, it can form a seal around the legs of the staple 2101'. As shown in FIG. 24D, a thickness of the first layer 2204 can be significantly reduced due to the melting, while the second layer 2205 can work in conjunction with the melting ORC to seal holes and gaps around the staple 2101'.

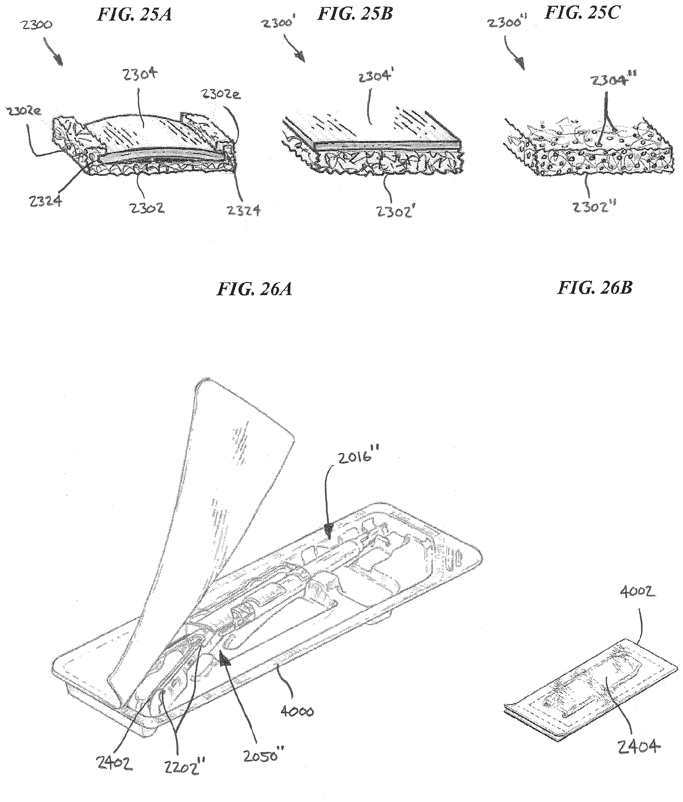

FIGS. 25A-25C provide additional, non-limiting techniques by which a hybrid adjunct material can be formed using both biologic material(s) and synthetic material(s). For example, as shown in FIG. 25A, a hybrid adjunct material 2300 having a snap-fit configuration can be formed in which the synthetic material, layer, or matrix 2302 is configured to have features for receiving the biologic material, layer, or matrix 2304. As shown, the synthetic layer 2302 includes opposed channels 2324 formed in outer edges 2302e of the layer 2302, and the biologic layer 2304 can be pliable such that it can be snap-fit into the synthetic layer 2302. In an alternative embodiment of a hybrid adjunct material 2300', which is illustrated in FIG. 25B, a synthetic material, layer, or matrix 2302' can be configured to have a biologic layer 2304' placed on top of it and then the two layers 2302', 2304' can be laminated together. A person skilled in the art will understand various techniques that can be performed to laminate the two layers 2302', 2304' together. In a further alternative embodiment of a hybrid adjunct material 2300", a synthetic material, layer, or matrix 2302" can have biologic material 2304" imbibed into the layer 2302", as shown in FIG. 25C. Other techniques capable of being used to combine synthetic material(s) and biologic material(s) to form a hybrid adjunct material can also be used without departing from the present disclosure.

A hybrid adjunct material that results from combining synthetic material(s) with biologic materials(s) as provided for herein can be associated with any and all of a cartridge assembly or lower jaw, a staple cartridge, and an anvil or upper jaw of an end effector using techniques known to those skilled in the art or otherwise provided for herein. For example, with respect to the hybrid adjunct materials 2300, 2300' of FIGS. 25A and 25B, the synthetic matrices 2302, 2302' can be pre-attached to any of a lower jaw, a staple cartridge, and an upper jaw and then the biologic layer 2304, 2304' can be attached thereto immediately prior to delivery. By way of further non-limiting example, such as for the embodiment of FIG. 25C, the hybrid adjunct material 2300", which includes both synthetic and biologic materials in the same layer, can be attached to the any of a lower jaw, a staple cartridge, and an upper jaw just prior to delivery of staples to a surgical site.

The components of hybrid adjunct materials can be associated with each other at any desired time, however, it can be preferable to add the biologic material(s) on site if the materials has a limited shelf-life, which many biologic materials do, particularly if they are not dry. Accordingly, as shown in FIGS. 26A and 26B, an attachment portion 2016" having an end effector 2050" with a synthetic material, layer, or matrix 2402 associated therewith can be packaged in a first container 4000, while a biologic material, layer, or matrix 2404 can be packaged in a second container 4002, separate from a first container 4000. As shown, the synthetic layer 2402 is pre-attached to the end effector 2050" by retention members 2202". Storing the biologic material 2404 in a separate, closed environment can be conducive to preserving its shelf-life. For example, the biologic material 2404 can be refrigerated prior to associating it with the synthetic material 2402 for subsequent deployment. Alternatively, the biologic material can be completely dried, which can also improve its shelf-life. In other embodiments, the synthetic material, layer, or matrix can be initially detached from an end effector, and can be packaged with or separate from either of the end effector or the biologic material. Prior to delivery of the synthetic and biologic materials to the surgical site, the two materials can be combined to form a hybrid adjunct material and can then be attached to the end effector for subsequent use at the surgical site. A person skilled in the art will understand other techniques that can be used to package the tool and the components of the hybrid adjunct material to preserve the shelf-life of any biologic materials without departing from the present disclosure.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination, e.g., electrodes, a battery or other power source, an externally wearable sensor and/or housing therefor, etc. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

In some embodiments, devices described herein can be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

One skilled in the art will appreciate further features and advantages of the invention based on the above-described embodiments. Accordingly, the invention is not to be limited by what has been particularly shown and described, except as indicated by the appended claims.

## Claims

1. A staple cartridge assembly for use with a surgical stapler, comprising:
a cartridge body (1052, 1054) having a plurality of staple cavities configured to seat staples therein;
a biologic tissue membrane (404, 404', 504, 604);
a synthetic layer (402, 402', 502, 602) associated with the membrane such that the synthetic layer is configured to provide structural integrity to the membrane so as to allow the membrane to be securely coupled to the cartridge body;
wherein the membrane (404, 404', 504, 604) and synthetic layer (402, 402', 502, 602) are configured to be securely attached to tissue by staples in the cartridge,
and
wherein the biologic tissue membrane (404, 404', 504, 604 2304) and the synthetic layer (402, 402', 502, 602, 2302) are configured to be snap-fit together.

2. The assembly of claim 1, wherein the synthetic layer (402, 402', 502, 602) has one or more openings (408) formed therein to allow biologic material of the biologic tissue membrane (404, 404', 504, 604) to pass through the synthetic layer (402, 402', 502, 602) and to tissue located adjacent to the synthetic layer.

3. The assembly of claim 1 or claim 2, further comprising at least one bracket (406, 406') configured to couple the biologic tissue membrane (404, 404', 504, 604) and the synthetic layer (402, 402', 502, 602) to the cartridge body (1052, 1054).

4. The assembly of claim 3, wherein the at least one bracket (406, 406') is coupled to an outer edge of the cartridge body and an outer edge of at least one of the membrane (404, 404', 504, 604) and the synthetic layer (402, 402', 502, 602).

5. The assembly of any preceding claim, further comprising a self-sealing port (520, 520') in fluid communication with at least one of the synthetic layer (402, 402', 502, 602) and the biologic tissue membrane (404, 404', 504, 604), the port being configured to permit delivery of patient-derived material to the membrane.

6. The assembly of any preceding claim, wherein the synthetic layer (402, 402', 502, 602) is permeable.

7. The assembly of any of claims 1 to 5, wherein the synthetic layer (402, 402', 502, 602) is non-permeable.

8. The assembly of any preceding claim, wherein the synthetic layer (402, 402', 502, 602) includes one or more protrusions (616) extending therefrom and configured to engage the biologic tissue membrane (404, 404', 504, 604).

## Patentansprüche

1. Klammermagazinanordnung zur Verwendung mit einer chirurgischen Klammervorrichtung, umfassend:
einen Magazinkörper (1052, 1054) mit einer Vielzahl von Klammerhohlräumen, die ausgelegt sind, darin sitzende Klammern aufzunehmen;
eine biologische Gewebemembran (404, 404', 504, 604);
eine synthetische Schicht (402, 402', 502, 602) in Zusammenhang mit der Membran, derart, dass die synthetische Schicht ausgelegt ist, der Membran strukturelle Integrität zu verleihen, damit die Membran sicher mit dem Magazinkörper verbunden werden kann;
wobei die Membran (404, 404', 504, 604) und die synthetische Schicht (402, 402', 502, 602) zur sicheren Befestigung an Gewebe durch Klammern im Magazin ausgelegt sind,
und
wobei die biologische Gewebemembran (404, 404', 504, 604, 2304) und die synthetische Schicht (402, 402', 502, 602, 2302) ausgelegt sind, ineinander einzurasten.

2. Anordnung nach Anspruch 1, wobei die synthetische Schicht (402, 402', 502, 602) eine oder mehrere darin ausgebildete Öffnungen (408) aufweist, damit biologisches Material der biologischen Gewebemembran (404, 404', 504, 604) durch die synthetische Schicht (402, 402', 502, 602) und zu Gewebe, das neben der synthetischen Schicht angeordnet ist, gelangen kann.

3. Anordnung nach Anspruch 1 oder Anspruch 2, ferner umfassend mindestens einen Haltebügel (406, 406'), der zur Verbindung der biologischen Gewebemembran (404, 404', 504, 604) und der synthetischen Schicht (402, 402', 502, 602) mit dem Magazinkörper (1052, 1054) ausgelegt ist.

4. Anordnung nach Anspruch 3, wobei der mindestens eine Haltebügel (406, 406') mit einem Außenrand des Magazinkörpers und einem Außenrand der mindestens einen Membran (404, 404', 504, 604) und der synthetischen Schicht (402, 402', 502, 602) verbunden ist.

5. Anordnung nach einem der vorhergehenden Ansprüche, ferner umfassend eine selbstdichtende Öffnung (520, 520') in Fluidverbindung mindestens mit der synthetischen Schicht (402, 402', 502, 602) und/oder der biologischen Gewebemembran (404, 404', 504, 604), wobei die Öffnung ausgelegt ist, die Abgabe von Material, das von einem Patienten stammt, an die Membran zu gestatten.

6. Anordnung nach einem der vorhergehenden Ansprüche, wobei die synthetische Schicht (402, 402', 502, 602) durchlässig ist.

7. Anordnung nach einem der Ansprüche 1 bis 5, wobei die synthetische Schicht (402, 402', 502, 602) undurchlässig ist.

8. Anordnung nach einem der vorhergehenden Ansprüche, wobei die synthetische Schicht (402, 402', 502, 602) einen oder mehrere Vorsprünge (616) aufweist, die sich von ihr erstrecken und ausgelegt sind, die biologische Gewebemembran (404, 404', 504, 604) in Eingriff zu nehmen.

## Revendications

1. Ensemble de cartouche d'agrafes à utiliser avec une agrafeuse chirurgicale, comprenant:
un corps de cartouche (1052, 1054) présentant une pluralité de cavités d'agrafe configurées de manière à contenir des agrafes dans celles-ci;
une membrane de tissu biologique (404, 404', 504, 604); et
une couche synthétique (402, 402', 502, 602) associée à la membrane de telle sorte que la couche synthétique soit configurée de manière à conférer une intégrité structurelle à la membrane de manière à permettre à la membrane d'être couplée fixement au corps de cartouche;
dans lequel la membrane (404, 404', 504, 604) et la couche synthétique (402, 402', 502, 602) sont configurées de manière à être attachées fixement au tissu par des agrafes dans la cartouche, et
dans lequel la membrane de tissu biologique (404, 404', 504, 604, 2304) et la couche synthétique (402, 402', 502, 602, 2302) sont configurées de manière à être emboîtées l'une avec l'autre.

2. Ensemble selon la revendication 1, dans lequel la couche synthétique (402, 402', 502, 602) comporte une ou plusieurs ouverture(s) (408) formée(s) dans celle-ci de manière à permettre à la matière biologique de la membrane de tissu biologique (404, 404', 504, 604) de passer à travers la couche synthétique (402, 402', 502, 602) et un tissu situé à proximité de la couche synthétique.

3. Ensemble selon la revendication 1 ou la revendication 2, comprenant en outre au moins une console (406, 406') configurée de manière à coupler la membrane de tissu biologique (404, 404', 504, 604) et la couche synthétique (402, 402', 502, 602) au corps de cartouche (1052, 1054).

4. Ensemble selon la revendication 3, dans lequel ladite au moins une console (406, 406') est couplée à un bord extérieur du corps de cartouche et à un bord extérieur d'au moins une parmi la membrane (404, 404', 504, 604) et la couche synthétique (402, 402', 502, 602).

5. Ensemble selon l'une quelconque des revendications précédentes, comprenant en outre un port auto-fermant (520, 520') en communication fluidique avec au moins une parmi la couche synthétique (402, 402', 502, 602) et la membrane de tissu biologique (404, 404', 504, 604), le port étant configuré de manière à permettre la délivrance d'une matière dérivée d'un patient à la membrane.

6. Ensemble selon l'une quelconque des revendications précédentes, dans lequel la couche synthétique (402, 402', 502, 602) est perméable.

7. Ensemble selon l'une quelconque des revendications 1 à 5, dans lequel la couche synthétique (402, 402', 502, 602) est imperméable.

8. Ensemble selon l'une quelconque des revendications précédentes, dans lequel la couche synthétique (402, 402', 502, 602) comporte une ou plusieurs saillie(s) (616) qui s'étend(ent) à partir de celle-ci et qui est/sont configurée(s) de manière à engager la membrane de tissu biologique (404, 404', 504, 604).
